# EUROPEAN PATENT SPECIFICATION

(11) **EP 4 346 677 B1**
(45) Date of publication and mention of the grant of the patent: **10.06.2026**
(21) Application number: 22744546.7
(22) Date of filing: 27.05.2022
(51) Int. Cl.: A61B 34/20, A61B 34/10, A61B 34/00, G16H 20/40, G16H 50/70, G16H 10/20, G16H 10/60, G16H 30/20, G16H 30/40, G16H 40/63, G16H 50/20, G16H 50/50, A61B 1/267, A61B 17/00, A61B 90/98

(54) **IMPROVED SYSTEMS OF NAVIGATING A MEDICAL DEVICE IN A BODY LUMEN USING FUZZY LOGIC COMBINED WITH DEVICE PARAMETERS, DIRECT USER INPUTS, AND DISTRIBUTED ANONYMIZED DATA**
VERBESSERTE SYSTEME ZUR NAVIGATION EINER MEDIZINISCHEN VORRICHTUNG IN EINEM KÖRPERLUMEN MIT FUZZY-LOGIK IN KOMBINATION MIT VORRICHTUNGSPARAMETERN, DIREKTEN BENUTZEREINGABEN UND VERTEILTEN ANONYMISIERTEN DATEN
SYSTÈMES AMÉLIORÉS DE NAVIGATION D'UN DISPOSITIF MÉDICAL DANS UNE LUMIÈRE CORPORELLE À L'AIDE D'UNE LOGIQUE FLOUE COMBINÉE AVEC DES PARAMÈTRES DE DISPOSITIF, DES ENTRÉES UTILISATEUR DIRECTES ET DES DONNÉES ANONYMISÉES DISTRIBUÉES

(30) Priority: 27.05.2021 US 202163194119 P
(43) Date of publication of application: 10.04.2024
(73) Proprietor: Covidien LP, Mansfield, MA 02048 (US)
(72) Inventor: KNUTSON, Nathan J., Plymouth, Minnesota 55441 (US); ROOTES, Matthew A., Plymouth, Minnesota 55441 (US); GUSTAFSON, Evan M., Plymouth, Minnesota 55441 (US); KOMP, John W., Boulder, Colorado 80301 (US); FRUSHOUR, Scott E.M., Boulder, Colorado 80301 (US)
(74) Representative: Zimmermann & Partner Patentanwälte mbB
(86) International application number: PCT/US2022/031464
(87) International publication number: WO 2022/251715

(56) References cited:
- WO-A1-2014/058838
- WO-A1-2017/196487
- WO-A1-2020/047051
- US-A1- 2017 143 428
- US-A1- 2018 060 524
- US-A1- 2019 380 792
- US-A1- 2020 268 472

## Description

### FIELD

The disclosed technology is generally related to guiding navigation of a medical device in a body lumen using fuzzy logic, medical device parameters, user input, and anonymous patient data.

### BACKGROUND

Current technology includes catheter systems that enable navigation of a catheter tip through a tortuous lumen of the body to a target. This technology involves the real-time movement of a catheter using medical images, e.g., computerized tomography (CT) images, of a target area of the body. Navigation may be presented as a recommended path based on the structure of the anatomy (e.g., an airway bronchiole structure) shown in the medical images. During navigation, however, the catheter systems may be unable to cause the distal portion of the catheter to reach all positions with an acceptable alignment with the target area.

US2018/060524A1 discloses systems, devices, and methods for planning a procedure for treatment of tissue in a patient's lungs. An exemplary method includes generating a three-dimensional (3D) model of the patient's lungs, displaying the 3D model of the patient's lungs, selecting a target location in the tissue of the patient's lungs as displayed on the 3D model, identifying a point on a pleural surface of the patient's lungs with access to the target location, determining an access path between the target location and the identified point on the pleural surface, calculating a risk of injury to intervening structures between the identified point on the pleural surface and the target location, based on the determined access path, and displaying the access path and the calculated risk of injury for the access path on the 3D model.

WO2014/058838A1 discloses a method wherein information extracted from sequential images captured from the perspective of a distal end of a medical device moving through an anatomical structure are compared with corresponding information extracted from a computer model of the anatomical structure. A most likely match between the information extracted from the sequential images and the corresponding information extracted from the computer model is then determined using probabilities associated with a set of potential matches so as to register the computer model of the anatomical structure to the medical device and thereby determine the lumen of the anatomical structure which the medical device is currently in. Sensor information may be used to limit the set of potential matches. Feature attributes associated with the sequence of images and the set of potential matches may be quantitatively compared as part of the determination of the most likely match.

WO2017/196487A1 discloses systems, devices, and methods for performing treatment along a lumen network, an exemplary method comprising receiving image data of a patient's lungs, mapping one or more luminal networks inside the patient's lungs based on the received image data, identifying a treatment target in the image data, determining a luminal pathway to the treatment target via at least one of the luminal networks, configuring treatment parameters for treatment of the treatment target and at least one of the luminal networks, navigating a tool inside at least one of the luminal networks to the treatment target, treating the treatment target with a primary treatment modality, and treating the luminal pathway of at least one of the luminal networks leading to or from the treatment target with a secondary treatment modality.

### SUMMARY

The invention is defined by appended claim 1.

The techniques of this disclosure generally relate to applying fuzzy logic to decision making and decision-guiding user interfaces to improve diagnostic yields and procedure efficiency. The techniques of this disclosure enable improved catheter system performance (e.g., diagnostic or therapy procedure time and success rate) through recommendations of best-practice navigation to the user, e.g., clinician or surgeon, based on the parameters of the physical performance of the medical device.

One aspect of the disclosure is directed to a method including receiving image data corresponding to a branched structure and receiving procedure information. The method also includes determining possible navigation paths for navigating a medical device within the branched structure using machine learning based on the image data and the procedure information; and displaying the possible navigation paths and controls for user selection of one of the possible navigation paths. Other implementations of this aspect include corresponding computer systems, apparatus, and computer programs recorded on one or more computer storage devices, each configured to perform the actions of the methods and systems described herein.

Implementations of this aspect of the disclosure may include one or more of the following features. The procedure information may include at least one of medical device physical characteristics, medical device physical behaviors, tissue information, previous procedure data, published peer-reviewed results, or path tortuosity. The method may include displaying maximum distances of the possible navigation paths. The device physical characteristics may include bend radius, stiffness for axial or transverse loads applied to a distal end portion of the medical device, multiplanar or single planar behavior, articulating section length, or number of articulation joints. The method may include determining a probability of success for navigation from all of the possible navigation paths for navigation based on physical characteristics of the medical device and tissue information; and displaying an indication of a probability for success for navigation for each path.

Implementations of the described techniques may include hardware, a method or process, or computer software on a computer-accessible medium, including software, firmware, hardware, or a combination of them installed on the system that, in operation, causes or cause the system to perform the actions. One or more computer programs can be configured to perform particular operations or actions by virtue of including instructions that, when executed by a data processing apparatus, e.g., a processor, cause the data processing apparatus to perform the actions.

A further aspect of the disclosure is directed to a method including receiving image data and receiving procedure information. The method also includes receiving previous clinician experience data. The method also includes determining possible navigation plans for navigating a medical device within a branched structure based on the image data and the procedure information; determining, for each possible navigation plan, a score for a difficulty to a clinician of carrying out the possible navigation plan using machine learning based on previous clinician experience data; and displaying the score for each possible navigation plan. Other implementations of this aspect include corresponding computer systems, apparatus, and computer programs recorded on one or more computer storage devices, each configured to perform the actions of the methods and systems described herein.

Implementations of this aspect of the disclosure may include one or more of the following features. The method may include tracking a position of the medical device during a procedure. The method may include determining that navigation of the medical device is unsuccessful based on the position of the medical device. The method may also include in response to determining that navigation of the medical device is unsuccessful, displaying a suggestion to use a different navigation plan. The method may include receiving previous procedure information. The method may include estimating, for each possible navigation plan, procedure time based on previous procedure information; and displaying the procedure time for each possible navigation plan. The method may include determining procedures similar to a current procedure. The method may include estimating the procedure time based on the previous procedure information for the current procedure and procedures similar to the current procedure.

The method may include determining a success rate or risk information for each navigation plan. The method may include displaying the success rate or the risk information for each navigation plan. The method may include determining positions of critical structures near each of the possible navigation plans. The method may include displaying warning indications for the positions of the critical structures. The method may include determining a success rate of a current clinician relative to success rates of other clinicians. The method may include displaying an indication of the success rate of the current clinician relative to success rates of other clinicians. The method may include receiving selection of a navigation plan from a current clinician.

The method may include determining that a current procedure is ended. The method may include in response to determining that the current procedure is ended, prompting the current clinician to input at least one outcome of the current procedure. The method may also include associating the navigation plan selected by the current clinician with the at least one outcome. The at least one outcome may be a complication during the current procedure or after the current procedure is ended. Implementations of the described techniques may include hardware, a method or process, or computer software on a computer-accessible medium, including software, firmware, hardware, or a combination of them installed on the system that, in operation, causes or cause the system to perform the actions. One or more computer programs can be configured to perform particular operations or actions by virtue of including instructions that, when executed by data processing apparatus, cause the apparatus to perform the actions.

A further aspect of the disclosure is directed to a navigation system including a human-controlled medical device. The navigation system also includes a position sensor coupled to the human-controlled medical device. The navigation system also includes a processor; and a memory having stored thereon a physical parameter and a behavior parameter of the human-controlled medical device, previous procedure information, and instructions, which, when executed by the processor, cause the processor to: receive preoperative image data; determine possible navigation plans within a branched structure based on the preoperative image data, the physical parameter, the behavior parameter of the human-controlled medical device, and previous procedure information; and display the possible navigation plans and controls for clinician selection of one of the possible navigation plans. Other implementations of this aspect include corresponding computer systems, apparatus, and computer programs recorded on one or more computer storage devices, each configured to perform the actions of the methods and systems described herein.

Implementations of this aspect of the disclosure may include one or more of the following features. The physical parameter may be in a look-up table stored on the memory and the behavior parameter may be based on previous performance data. The instructions, when executed by the processor, may cause the processor to determine that a current procedure is ended. The instructions, when executed by the processor, may cause the processor to in response to determining that the current procedure is ended, prompt a current clinician to input procedure performance information of the current procedure. The instructions, when executed by the processor, may cause the processor to store the current procedure performance information in association with the previous procedure information.

The instructions, when executed by the processor, may cause the processor to determine a control mode for the human-controlled medical device based on the preoperative image data. The instructions, when executed by the processor, may cause the processor to display a message requesting a current clinician to confirm whether to proceed in the control mode. The control mode may be a delicate tissue mode, a proximal lesion mode, an off-airway mode, or an ablation mode. The instructions, when executed by the processor, may cause the processor to prompt a current clinician to input patient information relevant to a current procedure. The instructions, when executed by the processor, may cause the processor to receive patient information. The instructions, when executed by the processor, may cause the processor to store the patient information in association with previous procedure information.

Implementations of the described techniques may include hardware, a method or process, or computer software on a computer-accessible medium, including software, firmware, hardware, or a combination of them installed on the system that, in operation, causes or cause the system to perform the actions. One or more computer programs can be configured to perform particular operations or actions by virtue of including instructions that, when executed by data processing apparatus, cause the apparatus to perform the actions.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of an electromagnetic navigation system in accordance with this disclosure;
FIG. 2 is a schematic diagram of a workstation configured for use with the system of FIG. 1;
FIGS. 3-6 illustrate user interfaces that present possible navigation paths based on input parameters related to navigation of an intraluminal medical device;
FIG. 7 illustrates a user interface for entering input parameters used to determine possible navigation paths; and
FIGS. 8 and 9 are flowcharts illustrating methods of guiding decision-making of a user based on various input parameters.

### DETAILED DESCRIPTION

Ideally, a catheter would be able to access all desired positions within an airway tree or other anatomical pathway. Reaching a desired position near a target, e.g., a lesion, alone is insufficient when sampling and/or treating tissue. Optimal alignment of the working channel is also critical to successful placement of a biopsy or therapy device at a target. However, during navigation, catheter systems may be unable to cause the distal portion of a catheter to achieve all positions with acceptable alignment with target. Some of the latest technology, including robotics, involves high manufacturing costs, large capital expenditures, and reprocessing of device components for limited uses to obtain favorable profit margins.

For catheter devices manufactured according to traditional catheter construction methods, performance approximates more complex devices in many cases but not in a universal way. In addition, catheter device performance may vary based on the skill sets, training, experience, and comfort of the users. Navigation is currently presented as a path based on the structure of the anatomy, such as airway bronchiole structure. Other variables which may impact how a procedure progresses includes tissue integrity, limitations of the catheter system devices (e.g., the scope and catheter), tool stiffnesses, nearby critical anatomical structures (e.g., the pleura or blood vessels greater than minimum size), and the goal of the procedure (e.g., biopsy or ablation).

The techniques of this disclosure generally relate to applying machine learning techniques, such as fuzzy logic (which mimics the logic of human thought), to decision-making and decision-guiding user interfaces to improve diagnostic yields and procedure efficiency. The techniques enable improved catheter system performance (e.g., diagnostic or therapy procedure time and success rate) through recommendations to a user, e.g., a clinician, of best-practice navigation based on the parameters of the physical performance of the medical device, e.g., minimum bend radius, articulating length, propensity to whip in rotation, pushability, torqueability, cross-sectional area, minimum or maximum diameter, and/or non-linear or asymmetric articulation behaviors.

Implementation details of such techniques, systems incorporating such techniques, and methods using the same are described below. However, these implementation details are merely examples of the disclosure, which may be embodied in various forms. Therefore, specific structural and functional details disclosed herein are not to be interpreted as limiting, but merely as a basis for the claims and as a representative basis for allowing one skilled in the art to variously employ this disclosure in virtually any appropriately detailed structure. While the example implementations described below are directed to the bronchoscopy of a patient's airways, those skilled in the art will realize that the same or similar devices, systems, and methods may also be used in other lumen networks, such as, for example, the vascular, lymphatic, and/or gastrointestinal networks.

Further, while described in connection with fuzzy logic, other artificial intelligence and/or machine learning methods may be employed to perform parameter-based path planning without departing from the scope of this disclosure. For example, neural networks, Markov decision processes, and/or others may be employed. Machine learning may include reinforcement learning, which involves rewarding desired behaviors and punishing negative behaviors. The reinforcement learning may assign positive values to the desired behaviors, e.g., medical procedure steps and/or actions that lead to a successful outcome, and may assign negative values to undesired behaviors, e.g., medical procedure steps and/or actions that lead to an unsuccessful outcome. The medical procedure steps and/or actions and the corresponding outcomes may be based on historical medical data.

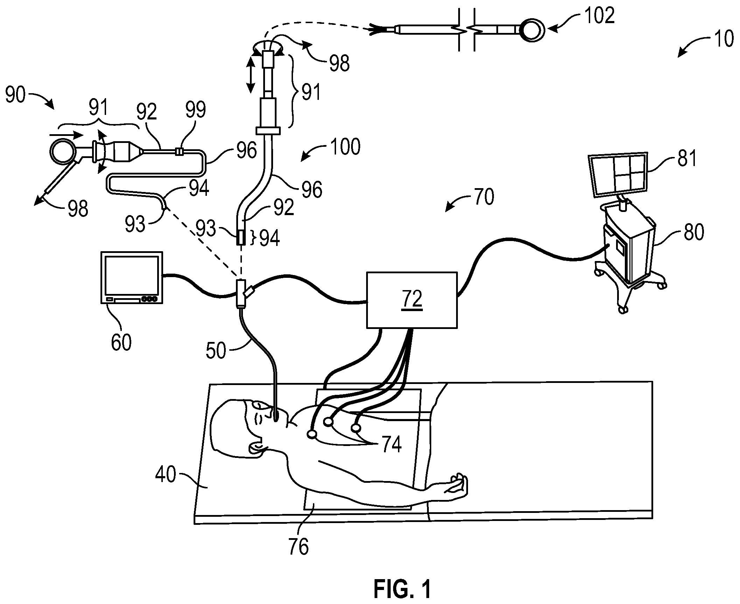

With reference to FIG. 1, an electromagnetic navigation (EMN) system 10 is provided in accordance with this disclosure. One such EMN system is the ELECTROMAGNETIC NAVIGATION BRONCHOSCOPY^{®} system currently sold by Covidien LP. Among other tasks that may be performed using the EMN system 10 are planning a pathway to target tissue, which may involve the fuzzy logic and machine learning techniques of this disclosure, navigating a positioning assembly to the target tissue, navigating a biopsy tool to the target tissue to obtain a tissue sample from the target tissue using the biopsy tool, digitally marking the location where the tissue sample was obtained, and placing one or more echogenic markers at or around the target.

EMN system 10 generally includes an operating table 40 configured to support a patient; a bronchoscope 50 configured for insertion through the patient's mouth and/or nose into the patient's airways; monitoring equipment 60 coupled to bronchoscope 50 for displaying video images received from bronchoscope 50; a tracking system 70 including a tracking module 72, a plurality of reference sensors 74, and an electromagnetic field generator 76; a workstation 80 including software and/or hardware used to facilitate pathway planning of this disclosure, identification of target tissue, navigation to target tissue, and digitally marking the biopsy location.

FIG. 1 also depicts two types of catheter guide assemblies 90, 100. Both catheter guide assemblies 90, 100 are usable with EMN system 10 and share a number of common components. Each catheter guide assembly 90, 100 includes a handle 91, which is connected to an extended working channel (EWC) 96. EWC 96 is sized for placement into the working channel of a bronchoscope 50. In operation, a locatable guide (LG) 92, including an electromagnetic (EM) sensor 94, is inserted into EWC 96 and locked into position such that EM sensor 94 extends a desired distance beyond a distal tip 93 of EWC 96. The location of EM sensor 94, and thus the distal end of EWC 96, within an electromagnetic field generated by electromagnetic field generator 76 can be derived by tracking module 72, and workstation 80. Catheter guide assemblies 90, 100 have different operating mechanisms, but each contain a handle 91 that can be manipulated by rotation and compression to steer distal tip 93 of LG 92 and EWC 96. Catheter guide assemblies 90 are currently marketed and sold by Covidien LP under the name SUPERDIMENSION^{®} Procedure Kits. Similarly, catheter guide assemblies 100 are currently sold by Covidien LP under the name EDGE^{™} Procedure Kits. Both kits include a handle 91, EWC 96, and LG 92.

As illustrated in FIG. 1, the patient is shown lying on operating table 40 with bronchoscope 50 inserted through the patient's mouth and into the patient's airways. Bronchoscope 50 includes a source of illumination and a video imaging system (not explicitly shown) and is coupled to monitoring equipment 60, e.g., a video display, for displaying the video images received from the video imaging system of bronchoscope 50.

Catheter guide assemblies 90, 100 including LG 92 and EWC 96 are configured for insertion through a working channel of bronchoscope 50 into the patient's airways (although the catheter guide assemblies 90, 100 may alternatively be used without bronchoscope 50). LG 92 and EWC 96 are selectively lockable relative to one another via a locking mechanism 99. A six degrees-of-freedom electromagnetic tracking system 70, or any other suitable positioning measuring system, is utilized for performing navigation, although other configurations are also contemplated. Tracking system 70 is configured for use with catheter guide assemblies 90, 100 to track the position of EM sensor 94 as it moves in conjunction with EWC 96 through the airways of the patient, as detailed below.

As shown in FIG. 1, electromagnetic field generator 76 is positioned beneath the patient. Electromagnetic field generator 76 and the plurality of reference sensors 74 are interconnected with tracking module 72, which derives the location of each reference sensor 74. One or more of reference sensors 74 are attached to the chest of the patient. The coordinates of reference sensors 74 are sent to workstation 80, which includes an application 81, which uses data collected by sensors 74 to calculate a patient coordinate frame of reference.

Also shown in FIG. 1 is a catheter biopsy tool 102 that is insertable into catheter guide assemblies 90, 100 following navigation to a target and removal of LG 92. Biopsy tool 102 is used to collect one or more tissue samples from the target tissue. Biopsy tool 102 is further configured for use in conjunction with tracking system 70 to facilitate navigation of biopsy tool 102 to the target tissue, tracking of a location of biopsy tool 102 as it is manipulated relative to the target tissue to obtain the tissue sample, and/or marking the location where the tissue sample was obtained.

Although navigation is detailed above with respect to EM sensor 94 being included in LG 92 it is also envisioned that EM sensor 94 may be embedded or incorporated within biopsy tool 102 where biopsy tool 102 may alternatively be utilized for navigation without need of LG 92 or the necessary tool exchanges that use of LG 92 requires.

During procedure planning or navigation, workstation 80 may utilize computed tomographic (CT) image data for generating and viewing the 3D model of the patient's airways, which may enable the identification of target tissue on the 3D model (automatically, semiautomatically or manually), and may allow for the selection of a pathway through the patient's airways to the target tissue. More specifically, the CT scans may be processed and assembled into a 3D volume, which is then utilized to generate the 3D model of the patient's airways. The 3D model may be presented on a display monitor associated with workstation 80, or in any other suitable fashion according to some aspects of this disclosure. Using workstation 80, various slices of the 3D volume and views of the 3D model may be presented and/or may be manipulated by a clinician to facilitate identification of a target, generation and display of possible pathways, and selection of a suitable pathway through the patient's airways to access the target. The 3D model may also show marks of the locations where previous biopsies were performed, including the dates, times, and other identifying information regarding the tissue samples obtained. These marks may also be selected as the target to which a pathway can be planned. Once selected, the pathway is saved for use during the navigation procedure.

During navigation, EM sensor 94, in conjunction with tracking system 70, enables tracking of EM sensor 94 and/or biopsy tool 102 as EM sensor 94 or biopsy tool 102 is advanced through the patient's airways.

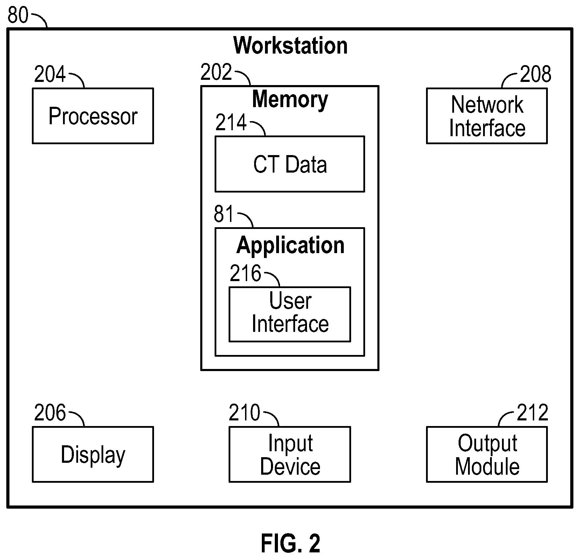

Turning to FIG. 2, there is shown a system diagram of workstation 80. Workstation 80 may include memory 202, processor 204, display 206, network interface 208, input device 210, and/or output module 212.

Memory 202 includes any non-transitory computer-readable storage media for storing data and/or software that is executable by processor 204 and which controls the operation of workstation 80. In one implementation, memory 202 may include one or more solid-state storage devices such as flash memory chips. As an alternative to or in addition to the one or more solid-state storage devices, memory 202 may include one or more mass storage devices connected to the processor 204 through a mass storage controller (not shown) and a communications bus (not shown).

Although the description of computer-readable storage media contained herein refers to solid-state storage, it should be appreciated by those skilled in the art that computer-readable storage media can be any available media that can be accessed by the processor 204. That is, computer-readable storage media may include non-transitory, volatile, non-volatile, removable, and/or non-removable media implemented in any method or technology for storage of information such as computer-readable instructions, data structures, program modules, or other data. For example, computer-readable storage media includes RAM, ROM, EPROM, EEPROM, flash memory or other solid state memory technology, CD-ROM, DVD, Blu-Ray or other optical storage, magnetic cassettes, magnetic tape, magnetic disk storage or other magnetic storage devices, or any other medium, which can be used to store the desired information and which can be accessed by workstation 80.

Memory 202 may store application 81 and/or CT/CBCT data 214. Application 81 may, when executed by processor 204, cause display 206 to present user interface 216. The application 81 may include a machine learning application or system as described herein. The user interface 216 may include one or more aspects of the user interfaces described below. Network interface 208 may be configured to connect to a network such as a local area network (LAN) consisting of a wired network and/or a wireless network, a wide area network (WAN), a wireless mobile network, a Bluetooth network, and/or the internet. Input device 210 may be any device by means of which a user may interact with workstation 80, such as, for example, a mouse, keyboard, foot pedal, touch screen, and/or voice interface. Output module 212 may include any connectivity port or bus, such as, for example, parallel ports, serial ports, universal serial busses (USB), or any other similar connectivity port known to those skilled in the art.

Though described in connection with a bronchoscopic implementation, this disclosure is not so limited and other motor-driven medical device or catheter systems, which may include one or more cameras, may be employed without departing from the scope of the disclosure. Similarly, the methods described herein may be executed in connection with one or more robotic- or manually-controlled and robotically-driven catheter systems. In such implementations, the bronchoscope may be replaced in full or in part by a catheter, catheter sleeve, steerable catheter, and other similar devices.

In aspects, various input variables may be stored and accessed by the system to determine possible navigation paths using machine learning techniques including a fuzzy logic system, and display them to the user or clinician for review and/or selection. The fuzzy logic system may include a knowledge database for storing the various input variables. And the various input variables may be used to define fuzzy rules, which may be used by a fuzzy inference engine to determine fuzzy outputs based on inputs conforming to predefined input variables. The input variables may include device physical behaviors. The device physical behaviors may include at least one of bend radius, stiffness for axial and transverse loads applied to the distal tip of the catheter, multiplanar or single planar behavior, articulating section length, and number of articulation joints. The device physical behaviors may be learned using machine learning or may be accessed from a look up table stored in memory.

In aspects, the input variables may include tool physical behaviors. The tool physical behaviors may include physical behaviors associated with at least one of a needle, a brush, forceps, a wire, and an ablation probe. The tool physical behaviors may be stored in an RFID incorporated in or on the medical device, e.g., positioned at a handle of the device, and/or may be received from the user in the form of planning information (e.g., one or more case goals). For example, the user may inform the system of the current tool through, for example, a suitable user interface. A manufacturer or other third party may manage a look up table of tool behaviors.

In aspects, the input variables may include tissue information. The tissue information may include indications of emphysema, the approximate age of tissue (which may be input according to a broad de-identified stratification of the original age data to comply with relevant health laws or regulations, e.g., the Health Insurance Portability and Accountability Act (HIPAA) Privacy Rules), or information regarding normal anatomy, abnormal anatomy, adhesions, or previous resections. The tissue information may include information inferred from preoperative imaging or information entered by the user during path planning. The tissue information may include anonymous data collected from previous cases or console data.

In aspects, the input variables may include inputs from previous case data or published peer reviewed results. For example, the input variables may be based on collected, past data that have led to a successful result. The previous case data may be obtained from recordings of cases or procedures by a catheter system console. The previous case data may also be obtained from user inputs including inputs from experienced account managers or input from current users, e.g., a surgeon or other clinician involved with the case.

In aspects, the input variables may include path tortuosity data. The path tortuosity data may include turn angles and the distance between turns. For example, the path tortuosity data may include a first turn having a first angle, e.g., 90 degrees, a second turn having a second angle, e.g., 193 degrees, and a distance, e.g., 2 cm, between the first turn and the second turn. The path tortuosity data may also indicate bends or turns that are unobtainable by the catheter system. The planning software may determine various path tortuosity data including one or more of: a number of turns, a distance between turns, which may include the length and angle of the airway segment, off-airway distances, and undersized airway distance. Then, the path tortuosity data may be compared to the physical and behavioral data of the medical device to determine possible paths to a target. For example, the path tortuosity data may be compared to the physical and behavioral data of the medical device to determine whether turns within candidate paths to the target are obtainable.

As will be appreciated, other factors in connection with the path tortuosity are the physical limitations of the catheter and tool combination. Certain catheters may have their bend radius greatly reduced by the tools to be deployed therethrough and thus the viability of a given navigation path may be affected by tool choice or catheter choice as well as the choice of their mutual deployment. The machine learning techniques of this disclosure may take into account these and other similar factors.

In aspects, the surgical navigation system may be based on a minimum amount of shared analysis between the user and the surgical navigation system to determine a navigation path to follow. In one implementation, two or more possible navigation paths may be presented on a display. The two or more possible navigation paths may be determined by a machine learning system based on one or more input variables. In some aspects, the possible navigation paths are determined based on various criteria, which may be incorporated into the machine learning, including airway criteria, e.g., "cross no blood vessels larger than 1mm" or "maximum needle excursion of 1.5 cm."

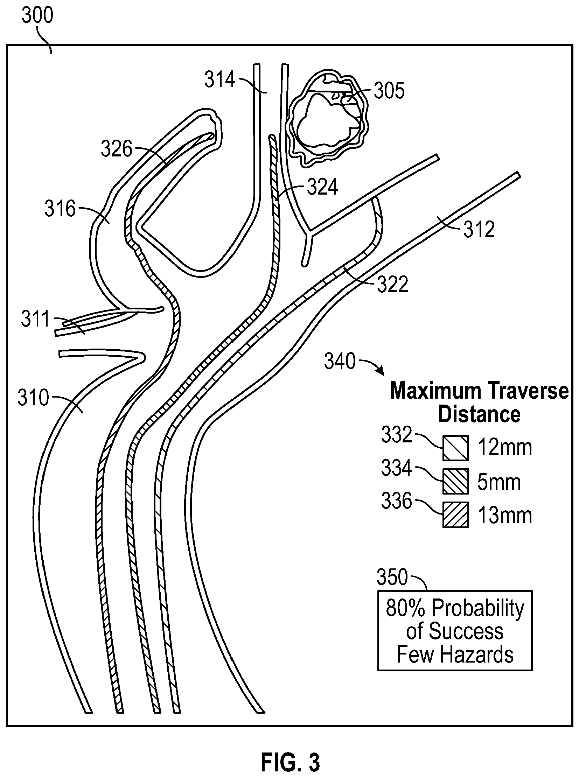

In aspects, the system may suggest, based on machine learning, nearby likely airway candidates for achieving alignment. FIG. 3 illustrates a user interface 300 showing a branched structure including a main branch 310 and branches 311, 312, 314, and 316. A first portion of possible navigation paths 312, 314, and 316 are displayed in the main branch 310 and second portions of the possible navigation paths 312, 314, and 316 are displayed in branches 322, 324, and 326, respectively. As shown in FIG. 3, the maximum traverse distances 340 from the distal portions of the possible navigation paths 322, 324, and 326 to the target 305 are displayed. In the example shown in FIG. 3, the maximum traverse distance values 12 mm, 5 mm, and 13 mm are displayed next to the path legend boxes 332, 334, and 336, respectively, which are displayed with a color or pattern that matches the color or pattern of the corresponding possible navigation paths 312, 314, and 316. In implementations, the path legend may use other methods to help a user or clinician distinguish between possible navigation paths and to provide relevant information regarding the possible navigation paths, such as corresponding traverse or travel distances for a medical device navigating through the possible navigation paths.

In aspects, the surgical navigation system may be based on a small amount of shared analysis between the user and the surgical navigation system to determine a navigation path to follow. The two or more possible navigation paths may be presented without weighting and without using machine learning to recommend one of the two or more possible navigation paths. Accordingly, the user can select one of the two or more possible navigation paths based on the user's skill and experience. In some aspects, the surgical navigation system may identify articulation requirements in order to navigate the medical device, e.g., catheter, according to each of the possible navigation paths. For example, during final positioning, the surgical navigation system may identify an articulation sequence to navigate the catheter to a position and/or orientation to successfully aim the tool at a target, such as a tissue structure.

In aspects, the surgical navigation system may be based on a medium amount of shared analysis between the user and the surgical navigation system to determine a navigation path to follow. In one implementation, two or more possible navigation paths may be determined using a machine learning algorithm and may be displayed with an indication of probabilities for or degrees of an acceptable or successful outcome. The two or more possible navigation paths may be determined alone or in combination with potential risks or hazards along with certain details on the identification of those hazards, which is illustrated in FIG. 3 as a text box 350 labelled "80% probability of success, fewest hazards" and positioned next to the legend box of the possible navigation path.

The surgical navigation system, utilizing machine learning such as fuzzy logic may prioritize possible navigation paths based on the input variables described herein including tissue information, physical or behavioral parameters of the intraluminal medical device, and/or previous user experience. For example, the surgical navigation system may take into consideration tissue condition, the arrangement of neighboring airways, proximity of airways to the target, and the feasibility of alignment of the intraluminal medical device (e.g., a catheter and a tool) with the target. In some aspects, the system may prioritize the feasibility of alignment over proximity of the airway to the target.

In analyzing probabilities for successful outcomes from among the possible navigation paths, the system may analyze the following considerations. As illustrated in FIG. 3, possible navigation path 322 is associated with a large airway, which is near the target 305 and accommodates a large catheter, but a catheter must make a sweeping bend to align with the target 305, and a tool must traverse 12 mm to reach the target 305. Possible navigation path 324 is associated with an airway nearest to the target 305 and thus has the shortest traverse distance. According to possible navigation path 324, a tool must exit the catheter at a sharp acute angle to strike the target 305. Possible navigation path 326 is associated with a small airway, which aligns with the target 305. According to possible navigation path 326, a tool has a straight approach to the target 305 once a catheter is positioned in the distal portion of the branch 316, but the tool must traverse a distance of 13 mm to reach the target 305. After an analysis of these considerations, the system may provide an indicator of the probability of success for a given navigation path 324, as illustrated in FIG. 3.

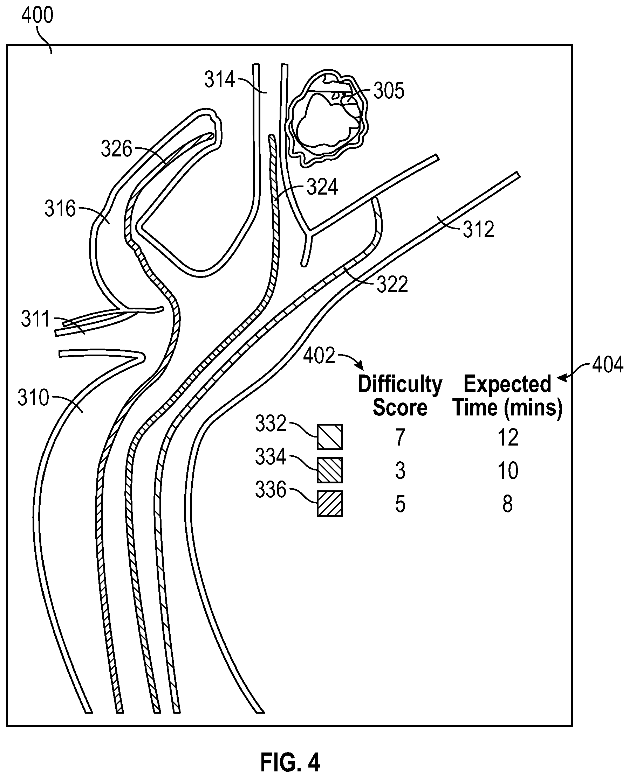

The surgical navigation system may also determine, using machine learning, and display a difficulty score for each of the two or more possible navigation paths. FIG. 4 shows a user interface 400 in which a difficulty score 402 is displayed next to each of the legend boxes 332, 334, and 336.

The probability of success score or difficulty for each of the possible paths may be indicated by the color of the displayed path. For example, a first possible path may be colored red to indicate that the first possible path has the lowest probability of success score, a second possible path may be colored green to indicate that the first possible path has the highest probability of success score, and a third possible path may be colored yellow to indicate that the third possible path has a probability of success score between the probability of success scores for the second and third possible paths.

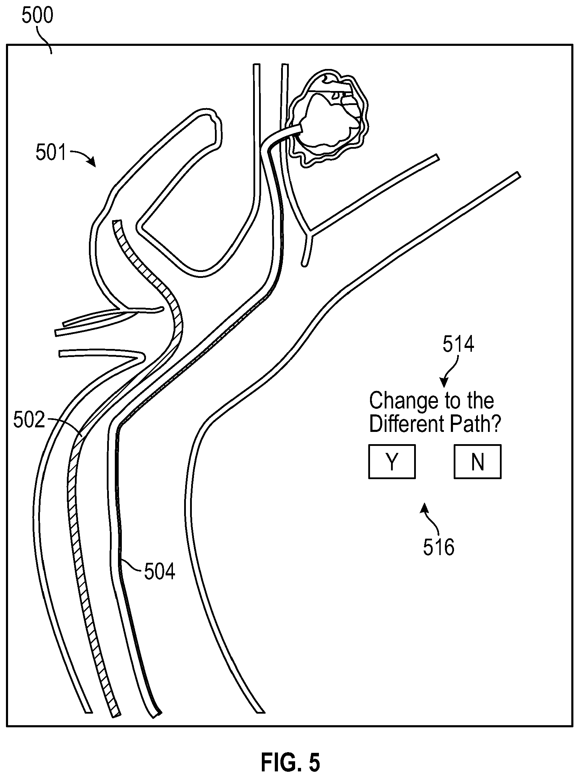

In aspects, the system may determine possible paths that avoid conflicts. One example of a conflict may include a sharp turn, which cannot be navigated by the catheter based on the input characteristics of the catheter. For example, the input characteristic of the catheter is that it cannot be articulated in such a way as to make the sharp turn. In aspects, the surgical navigation system may make suggestions in real time while information is being gathered during the procedure. For example, as illustrated in FIG. 5, if the surgical navigation system determines that a turn 501 has not been successfully navigated by the catheter in multiple attempts based on catheter position feedback 502, the system may suggest a different path 504. The user interface 500 may display the message "Change to the different path" 514, in which case the user may select one of buttons 516 to switch to navigation path 504.

In aspects, relevant patient data may be communicated from an electronic health record (EHR) system or a picture archiving and communication system (PACS) to the surgical console. All patient data may be de-identified, roughened, and/or anonymized prior to being communicated to the surgical console to allay privacy concerns.

In aspects, the system may display the expected procedure time to the user. As illustrated in FIG. 4, an expected time 404 may be displayed next to each of the legend boxes 332, 334, and 336. The expected time may be computed based on a de-identified, roughened, and/or anonymized set of other patient data including image data and actual procedure times. Alternatively, or additionally, the system may display the following or similar textual message: "Based on 2,371 other procedures similar to the current procedure (biopsy, poor lung tissue condition, peripheral lower lobe) the median procedure time is 15 minutes with a 25^{th} percentile time of 10 minutes and a 75^{th} percentile time of 20 minutes."

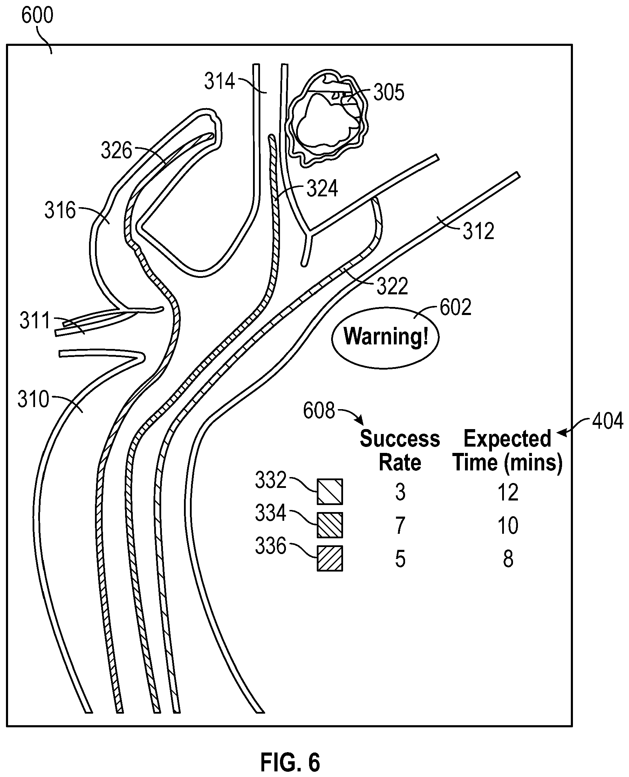

In aspects, the system may display relative success rates or risks for the top three possible paths through a branched structure of airways. For example, the system may display probability of pneumothorax, blood vessel puncture, or any other risks associated with the procedure. The system may determine positions of critical structures and display the positions of the critical structures. As illustrated in FIG. 6, the system may further present user warnings 602 as the intraluminal medical device nears positions of critical structures.

In aspects, the system may implement a game among users using the result of the analysis of users' performance data. For example, the system may provide feedback to a user on success rates 608 relative to the user's peers, as illustrated in FIG. 6. The feedback may be provided in the form of a game, in which, for example, points are assigned to users based on their cumulative performance, e.g., success rate, and displayed to the users to encourage competition among users, and thus improve overall success rates.

In aspects, the system may gather data after the procedure. For example, the system may prompt the user to input information regarding the performance of the procedure. The performance information may be self-reported by the surgical navigation system to a medical database. The performance information may include a relationship between the expected plan and the observed outcome. The performance information may be used as a closed-loop input to the surgical navigation system. In aspects, the EMR or PACS may be updated to include intra-operative and post-operative information. For example, the EMR or PACS may be updated to include intra-operative or post-operative information relating to any complications. The intra-operative and post-operative information may be updated at predetermined times, e.g., during the procedure, one week after the procedure, one month after the procedure, one year after the procedure, or at any combination of times.

In aspects, the surgical navigation system may incorporate position sensing information to access natural orifices. The manufacturer of the surgical navigation system may store known structural parameters, behavioral parameters, and/or collected performance data of the access catheter or device in memory. The parameters and/or performance data may be organized in a look-up table. The system may include computer or data processing devices for collecting and anonymizing data from previous procedures. After the procedure, the surgical navigation system may store procedure data and/or prompt the user to input data that may be used for future similar procedures. For example, the surgical navigation system may store an actual case time, which may be entered by a user or may be automatically stored by the system in system memory or in a secured server.

The system may present questions relating to the procedure and enable the user to input an answer, such as a binary yes/no answer, a nonbinary answer (e.g., a scale from 1 to 5), or a textual answer, or a combination of two or more of these answer types. For example, the questions may include one or more of the following questions: "Did you accomplish your navigation goals for this case?," "Did you accomplish your alignment or target biopsy goals for this case?," "How do you feel the system-generated assistance performed today?," or "Any observed complications as of the time of this entry?"

During planning or during a procedure, the user interface may prompt the user based on preoperative imaging. For example, the system may analyze preoperative imaging, and, based on that analysis, the user interface may display the following message including a prompt as follows: "From preoperative imaging, it appears the patient displays compromised lung tissue structure. Would you like to proceed in **delicate tissue mode?"** The system may analyze the preoperative imaging based on computer-aided diagnosis methods that perform image processing on preoperative imaging to recognize possible issues with tissue structures. The system may analyze the preoperative imaging using machine learning-based techniques.

For procedures involving human-controlled or human-actuated devices, the surgical navigation system may recommend a control mode. The control modes may include a delicate tissue mode, a proximal lesion mode, an off-airway mode, an ablation mode, or any combination of these modes. In the delicate tissue mode, the surgical navigation system may prioritize various navigation strategies. The surgical navigation system may prioritize: straight-on navigation, larger airways for the first portion of the path (e.g., 90%), and routes which do not require large changes in direction during navigation (e.g., a first angle of 90 degrees and a second angle of 270 degrees). The surgical navigation system may identify a particular tool, a particular set of tools, or an order in which a set of tools are used in a procedure. The proximal lesion mode may, for example, prioritize paths which result in greater than three points of estimated contact by a catheter with nearby airway walls, and may prioritize paths which place the catheter tip proximal to the lesion to facilitate performing a procedure on the lesion.

The control modes may include an off-airway mode. The off-airway mode may be recommended in cases where following the airway route would lead to a decreased chance of success. Additionally, or alternatively, the control modes may include an ablation mode, by which the surgical navigation system utilizes fuzzy logic and/or machine learning to determine and recommend possible paths and/or plans for successfully completing an ablation procedure. The surgical navigation system may utilize user inputs leading to hard-coded recommendations for the user and/or learned and/or taught recommendations for the user. The surgical navigation system may prompt a user for a variety of inputs regarding the patient, e.g., medical or surgical history information, via a user interface. The inputs may be acquired during planning or as optional entries during navigation and used in the machine learning techniques (e.g., fuzzy logic algorithms) of this disclosure.

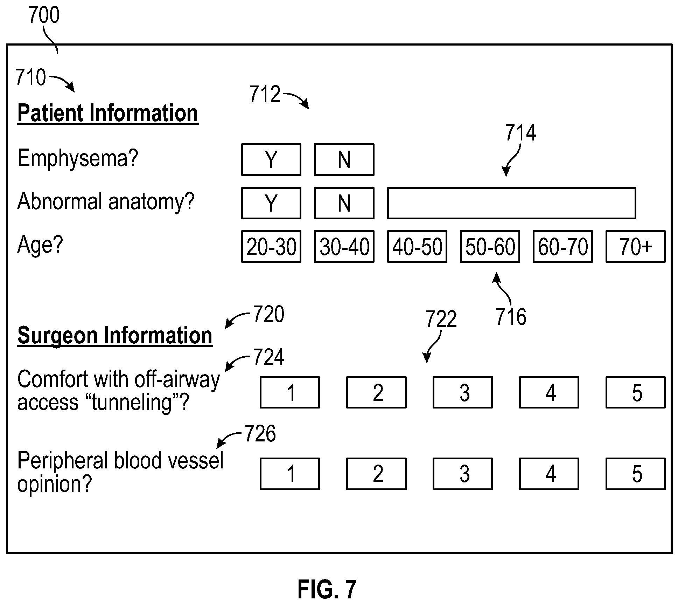

For example, before a procedure starts, the system may display user interface 700 of FIG. 7. The system may prompt a user to input information regarding emphysema or comorbidities. Alternatively, the system may prompt a user to input information regarding a condition relevant to the procedure. The system may prompt the user to input information regarding abnormal anatomy, such as missing tissue, adhesions, or large or broadly dispersed lesions. For example, under the "Patient information" heading 710 of the user interface 700, the questions "Emphysema?" and "Abnormal anatomy?" are displayed with buttons 712, which may be selected by the user to answer the questions.

The user interface 700 may further include a data field 714, in which text may be entered by the user to identify the abnormal anatomy. The system may prompt the user to input information regarding previous resections. The system may prompt the user to input the approximate age of the patient, e.g., the decade in which the patient's age falls. For example, as illustrated in FIG. 7, one of buttons 716 may be selected to input the age of the patient, which may be used to access anonymized health, medical, or surgical information of individuals at and/or approximately at the age of the patient, and may be used a machine learning system of this disclosure.

To build a knowledge database and implement machine learning, the surgical navigation system may prompt a user to input information relating to the user via a user interface. For example, the system may display user interface 700, in which the user, e.g., a surgeon, may enter information regarding the user in relation to a procedure under the heading "Surgeon Information" 720. The user information may include the user's experience or comfort level with performing a particular procedure or portion of the procedure. For example, the surgical navigation system may prompt a user to input information regarding the user's comfort with off-airway access "tunneling" 724 to the target. The user may input more specific information regarding the user's comfort level for the location or length of the off-airway access tunneling, e.g., the user's comfort levels for the off-airway distances.

The user information may include the user's medical opinion. The user's medical opinion may include the user's medical opinion on the patient's anatomy, such as the peripheral blood vessels 726. The user's medical opinion may indicate the level of care needed to successfully complete the procedure. The artificial intelligence, machine learning, or fuzzy logic methods of this disclosure may then be used to process the patient information 710, the surgeon information 720, and/or anonymized patient and surgeon information to determine possible and/or recommended surgical plans and/or paths, and to display the possible and recommended surgical plans and/or paths to the user.

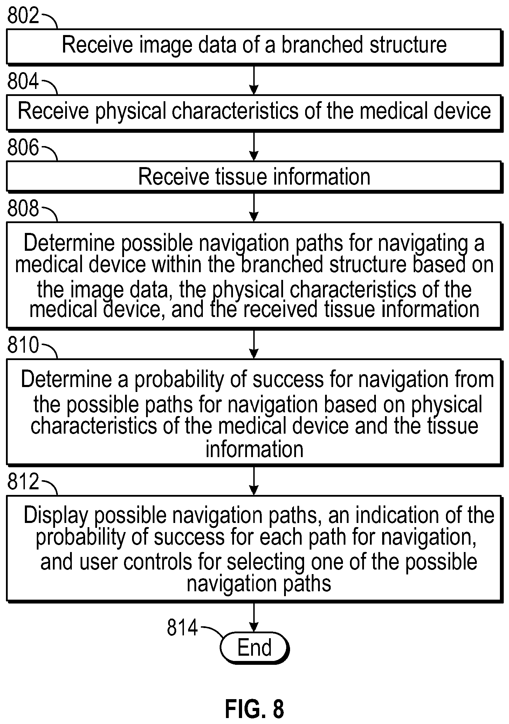

FIG. 8 is a flowchart illustrating a method of guiding decision-making of a user relating to navigation of an intraluminal medical device based on various input parameters. At block 802, image data of a branched structure in a patient's body is received. At block 804, physical characteristics of the intraluminal medical device are received. The medical device physical characteristics may include bend radius, stiffness for axial or transverse loads applied to a distal end portion of the medical device, multiplanar or single planar behavior, articulating section length, and/or the number of articulation joints. The medical device physical characteristics may be stored and updated in a knowledge database of a machine learning algorithm, such as a fuzzy logic algorithm described herein.

At block 806, tissue information is received. The tissue information may include indications of abnormal or diseased tissue or tissue with a condition, e.g., emphysema, the approximate age of tissue (which may be input according to a broad de-identified stratification of the original age data to comply with relevant health laws or regulations, e.g., the Health Insurance Portability and Accountability Act (HIPAA) Privacy Rules), and/or information regarding normal anatomy, abnormal anatomy, adhesions, or previous resections. The tissue information may include information inferred from preoperative imaging or information entered by the user during path planning. The tissue information may include anonymous data collected from previous cases or console data.

In aspects, other or different procedure information may be received and used in a machine learning system of this disclosure. The procedure information may include medical device physical behaviors, previous procedure data, published peer-reviewed results, and/or path tortuosity.

At block 808, possible navigation paths for navigating a medical device within the branched structure are determined based on the image data, the physical characteristics of the medical device, and the received tissue information. At block 810, a probability for success of navigation for the possible paths for navigation is determined based on physical characteristics of the intraluminal medical device and the tissue information. Then, before ending at block 814, possible navigation plans, an indication of the probability of success of navigation, and user controls for selecting one of the possible navigation plans are displayed at block 812.

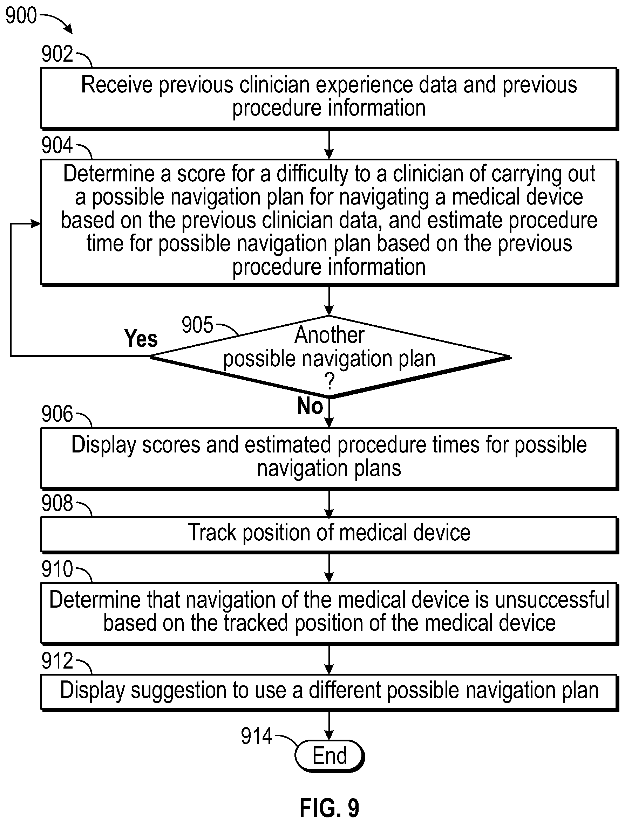

FIG. 9 is a flowchart illustrating another method of guiding decision-making of a user relating to navigation of an intraluminal medical device based on various inputs incorporated into a fuzzy logic algorithm. While this example identifies specific inputs, one or more of the inputs described above may be incorporated into the fuzzy logic system. At block 902, previous clinician experience data and previous procedure information is received. At block 904, a score for a difficulty to a clinician of carrying out a possible navigation plan for navigating a medical device is determined based on the previous clinician data. The score may be determined according to the following fuzzy logic algorithm. Previous clinician experience data and previous clinician procedure information are applied to a fuzzifier, which uses a knowledge database to obtain fuzzy values. The knowledge database may store knowledge on all the input-output fuzzy relationships and may have a membership function which defines the input variables to the fuzzy rule database and the output variables. Then, the fuzzy values are applied to a decision-making algorithm, which simulates human decisions based on the fuzzy values by performing approximate reasoning. The decision-making algorithm may be a rules-based inference engine, which generates fuzzy outputs based on information from the fuzzy rule database. The database may include fuzzy rules assigning a relationship between fuzzy input and fuzzy output. The fuzzy logic algorithm may also include a defuzzifier, which converts the fuzzy values output from the inference engine into crisp values

Also, a procedure time for a possible navigation plan may be estimated based on the previous procedure information. At block 905, the method 900 determines whether there is another possible navigation plan. If there is another possible navigation plan, block 904 is performed for the other possible navigation plan. If there is not another possible navigation plan, scores and estimated procedure times for possible navigation plans are displayed at block 906. At block 908, the position of the medical device is tracked. At block 910, it is determined that navigation of the medical device is unsuccessful based on the tracked position of the medical device. Then, before ending at block 914, a suggestion to use a different possible navigation plan, which may be determined using machine learning, is displayed at block 912.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

## Claims

1. A navigation system (10) comprising:
a human-controlled medical device (100);
a position sensor (94) coupled to the human-controlled medical device;
a processor (204); and
a memory (202) having stored thereon a physical parameter and a behavior parameter of the human-controlled medical device, previous procedure information, and instructions, which when executed by the processor, causes the processor to:
receive preoperative image data;
determine possible navigation plans within a branched structure based on the preoperative image data, the physical parameter, the behavior parameter of the human-controlled medical device, and previous procedure information; and
display the possible navigation plans and controls for clinician selection one of the possible navigation plans, wherein the physical parameter is in a look-up table stored on the memory and the behavior parameter is based on previous performance data.

2. The navigation system of claim 1, wherein the instructions, when executed by the processor, further cause the processor to:
determine that a current procedure is ended;
in response to determining that the current procedure is ended, prompt a current clinician to input procedure performance information of the current procedure; and
store the current procedure performance information in association with the previous procedure information.

3. The navigation system of claim 1, wherein the instructions, when executed by the processor, further cause the processor to:
determine a control mode for the human-controlled medical device based on the preoperative image data; and
display a message requesting a current clinician to confirm whether to proceed in the control mode.

4. The navigation system of claim 3, wherein the control mode is a delicate tissue mode, a proximal lesion mode, an off-airway mode, or an ablation mode.

5. The navigation system of claim 1, wherein the instructions, when executed by the processor, further cause the processor to:
prompt a current clinician to input patient information relevant to a current procedure;
receive patient information; and
store the patient information in association with previous procedure information.

## Patentansprüche

1. Navigationssystem (10), umfassend:
eine von Menschen gesteuerte medizinische Vorrichtung (100);
einen Positionssensor (94), der an die von Menschen gesteuerte medizinische Vorrichtung gekoppelt ist;
einen Prozessor (204); und
einen Speicher (202)
auf dem ein physischer Parameter und ein Verhaltensparameter der von Menschen gesteuerten medizinischen Vorrichtung, vorherige Prozedurinformationen und Anweisungen gespeichert sind, die bei Ausführung durch den Prozessor den Prozessor hierzu veranlassen:
Empfangen präoperativer Bilddaten;
Bestimmen möglicher Navigationspläne innerhalb einer verzweigten Struktur basierend auf den präoperativen Bilddaten, dem physischen Parameter, dem Verhaltensparameter der von Menschen gesteuerten medizinischen Vorrichtung und vorherigen Prozedurinformationen; und
Anzeigen der möglichen Navigationspläne und Steuerungen zur Klinikerauswahl eines der möglichen Navigationspläne, wobei sich der physische Parameter in einer Nachschlagetabelle befindet, die in dem Speicher gespeichert ist und der Verhaltensparameter auf vorherigen Leistungsdaten basiert.

2. Navigationssystem nach Anspruch 1, wobei die Anweisungen bei Ausführung durch den Prozessor den Prozessor ferner hierzu veranlassen:
Bestimmen, dass eine aktuelle Prozedur beendet ist;
in Reaktion auf das Bestimmen, dass die aktuelle Prozedur beendet ist, Auffordern eines aktuellen Klinikers, Prozedurleistungsinformationen der aktuellen Prozedur einzugeben; und
Speichern der aktuellen Prozedurleistungsinformationen in Verbindung mit den vorherigen Prozedurinformationen.

3. Navigationssystem nach Anspruch 1, wobei die Anweisungen bei Ausführung durch den Prozessor den Prozessor ferner hierzu veranlassen:
Bestimmen eines Steuerungsmodus für die von Menschen gesteuerte medizinische Vorrichtung basierend auf den präoperativen Bilddaten; und
Anzeigen einer Nachricht, die einen aktuellen Kliniker auffordert zu bestätigen, ob in dem Steuerungsmodus weitergearbeitet werden soll.

4. Navigationssystem nach Anspruch 3, wobei der Steuerungsmodus ein Empfindliches-Gewebe-Modus, ein Proximale-Läsion-Modus, ein luftwegexterner Modus oder ein Ablationsmodus ist.

5. Navigationssystem nach Anspruch 1, wobei die Anweisungen bei Ausführung durch den Prozessor den Prozessor ferner hierzu veranlassen:
Auffordern eines aktuellen Klinikers, Patienteninformationen einzugeben, die für eine aktuelle Prozedur relevant sind;
Empfangen von Patienteninformationen; und
Speichern der Patienteninformationen in Verbindung mit vorherigen Prozedurinformationen.

## Revendications

1. Système de navigation (10) comprenant :
un dispositif médical sous commande humaine (100) ;
un capteur de position (94) couplé au dispositif médical sous commande humaine ;
un processeur (204) ; et
une mémoire (202)
dans laquelle sont mémorisés un paramètre physique et un paramètre de comportement du dispositif médical à commande humaine, des informations d'interventions antérieures, et des instructions qui, lorsqu'elles sont exécutées par le processeur, amènent le processeur à :
recevoir des données d'images préopératoires ;
déterminer des plans de navigation possibles au sein d'une structure ramifiée en fonction des données d'images préopératoires, du paramètre physique, du paramètre de comportement du dispositif médical à commande humaine, et d'informations d'interventions antérieures ; et
afficher les plans de navigation et les commandes possibles pour la sélection par le clinicien de l'un des plans de navigation possibles, le paramètre physique se trouvant dans une table de consultation stockée dans la mémoire et le paramètre de comportement étant basé sur des données de performances antérieures.

2. Système de navigation selon la revendication 1, dans lequel les instructions, lorsqu'elles sont exécutées par le processeur, amènent en outre le processeur à :
déterminer qu'une intervention en cours est terminée ;
en réponse à la détermination de la fin de l'intervention en cours, inviter un clinicien actuel à entrer des informations de performances d'intervention en cours ; et
stocker les informations de performances d'intervention en cours en association avec les informations d'interventions antérieures.

3. Système de navigation selon la revendication 1, dans lequel les instructions, lorsqu'elles sont exécutées par le processeur, amènent en outre le processeur à :
déterminer un mode de commande pour le dispositif médical à commande humaine en fonction des données d'images préopératoires ; et
afficher un message demandant à un clinicien actuel de confirmer s'il faut passer ou non en mode commande.

4. Système de navigation selon la revendication 3, dans lequel le mode commande est un mode tissu délicat, un mode lésion proximale, un mode hors voies aériennes ou un mode ablation.

5. Système de navigation selon la revendication 1, dans lequel les instructions, lorsqu'elles sont exécutées par le processeur, amènent en outre le processeur à :
inviter un clinicien actuel à entrer des informations patient pertinentes pour une intervention en cours ;
recevoir des informations patient ; et
stocker les informations patient en association avec les informations d'interventions antérieures.
